# EUROPÄISCHE PATENTSCHRIFT

(11) **EP 2 149 387 B1**
(45) Veröffentlichungstag und Bekanntmachung des Hinweises auf die Patenterteilung: **12.06.2019**
(21) Anmeldenummer: 09164877.4
(22) Anmeldetag: 08.07.2009
(51) Int. Cl.: A61M 25/10, A61M 25/00

(54) **Ballonkatheter und Verfahren zu dessen Herstellung**
Balloon catheter and method for its production
Cathéter à ballonnet et son procédé de fabrication

(30) Priorität: 01.08.2008 DE 102008040914
(43) Veröffentlichungstag der Anmeldung: 03.02.2010
(73) Patentinhaber: BIOTRONIK AG, 8180 Bülach (CH)
(72) Erfinder: Brunner, Arthur, 8916, Jonen (CH); Blaser, Adrian, 8032 Zürich (CH); Wesselmann, Matthias, 8455 Rüdlingen (CH); Bachmann, Patrice, 8408, Winterthur (CH)
(74) Vertreter: Keck, Hans-Georg

(56) Entgegenhaltungen:
- DE-A1- 2 847 633
- US-A- 4 994 072
- US-A1- 2003 032 921
- US-A1- 2003 180 488

## Beschreibung

Die Erfindung betrifft ein Verfahren zur Herstellung eines Ballonkatheters.

Im Oberbegriff des Anspruches 1 ist die Grundkonstruktion von Ballonkathetern angegeben, wie sie beispielsweise aus den Druckschriften US 5,522,882 A und US 7,217,278 B2 bekannt ist. So weisen Ballonkatheter, wie sie etwa für das Aufweiten von krankhaft verengten Gefäßen im Körper oder für das Setzen von Gefäßwandstützen - sogenannter "Stents" - eingesetzt werden, einen Außenschaft mit einem distalen Ende und einen darin unter Bildung einer ringförmigen Fluidleitung angeordneten Innenschaft auf, der über das distale Ende des Außenschaftes hinaussteht. Am distalen Ende des Katheters ist ein Ballon mit einer ersten Befestigungszone an seinem proximalen Ende fluiddicht auf dem distalen Endbereich des Außenschaftes und mit einer zweiten Befestigungszone an seinem distalen Ende fluiddicht auf dem distalen Endbereich des Innenschaftes befestigt. Zwischen diesen Befestigungszonen ist der Ballon in undilatiertem Zustand in Längsfalten gelegt, damit sein Außendurchmesser in diesem Zustand möglichst gering ist. Dies ist Voraussetzung dafür, dass der Ballonkatheter mit dem Ballon am distalen Ende auch durch enge Gefäße oder stark gekrümmte Gefäßbereiche vorgeschoben werden kann. Nach der Platzierung des Ballons an seinem Einsatzort kann dann über die zwischen Innen- und Außenschaft gebildete ringförmige Fluidleitung ein Fluid unter Druck eingebracht und der Ballon dilatiert werden. Dabei entfalten sich die Längsfalten in Peripherrichtung unter starker Durchmesservergrößerung des Ballons.

US 4 994 072 A offenbart darüber hinaus die Merkmale des Oberbegriffs von Anspruch 1.

Die Ballons herkömmlicher Ballonkatheter weisen aufgrund ihrer Herstellungsart und Konstruktion Nachteile auf, die aus dem folgenden Abriss des Produktionsprozesses deutlich werden. So werden Ballons in der Regel aus einem plastisch reckbaren Kunststoffröhrchen mit einem Außendurchmesser von beispielsweise 2,1 mm und einem Lumen-Durchmesser von beispielsweise 1,5 mm hergestellt. Die Wandstärke dieses Röhrchens beträgt also 0,3 mm. Seine Enden werden in eine Haltevorrichtung eingespannt, worauf das Lumen mit einem Fluiddruck beaufschlagt wird. Zwischen den Einspannpunkten wird das Werkstück aufgeblasen und das Wandmaterial drastisch gereckt, so dass ein im Wesentlichen zylindrischer Ballon mit einer Wandstärke beispielsweise 0,03 mm entsteht. Von den eingespannten Enden des Ballonrohlings aus verringert sich die Wandstärke über die Konen an den beiden Enden des Ballons zur Mantelwand hin also etwa um den Faktor 10.

In einem weiteren Bearbeitungsschritt werden die Enden noch kalibriert und beispielsweise auf einen Außendurchmesser von 1,8 mm sowie einen Lumen-Durchmesser von 1,6 mm gebracht. Die Wandstärke beträgt dann noch 0,1 mm und beträgt somit immer noch mehr als das Dreifache der Wandstärke im zylindrischen Teil des Ballonrohlings.

Werden nun derart hergestellte Ballons mit ihren Enden auf dem Außen- bzw. Innenschaft des Katheters befestigt und für den undilatierten Zustand in Längsfalten gelegt, so tragen die eine deutlich größere Wandstärke aufweisenden Enden und Konen des Ballons deutlich stärker auf als der sehr dünnwandige Zylindermantel des Ballons. Das gefaltete Ballonprofil ist also an den die Ballonschultern bildenden Konen um die Befestigungszonen am größten. Entsprechend ist auch die Steifigkeit des Ballons dort am stärksten ausgebildet. Damit behindern diese stark auftragenden Endbereiche des Ballons ein Einführen des Katheters in enge Gefäße. Die Steifigkeit der in Falten gelegten Ballonkonen erschwert darüber hinaus das Führen des Ballonkatheters um enge Krümmungen oder Abzweigungen von Gefäßen. Schließlich ist es bei der Herstellung des Ballonkatheters selbst schwierig, die eine höhere Wandstärke aufweisenden Konenbereiche zu falten.

Die vorstehend erwähnte US 5,522,882 A offenbart ein Stent-Positionierungs-System mit einem Katheter, bei dem der dilatierbare Ballon stufenförmig verlaufende Enden aufweist. Damit sollen axial vor und nach dem auf dem Katheter positionierten Stent keine Konenabschnitte des Ballons vorhanden sein. Dies wird durch hülsenartige Aufsatzstücke erreicht, die auf den Ballon-Endbereichen unmittelbar an den Stent angrenzen, so dass beim Dilatieren des Ballons sich die Konen im Wesentlichen als radiale Ringstufen ausprägen. Die Problematik der unterschiedlichen Wandstärken und der Faltenlegung bei herkömmlichen Ballons ist in dieser Druckschrift nicht angesprochen.

Die US 7,217,278 B2 lehrt eine aufwändige Nachbearbeitung von Ballonrohlingen, indem nach dem Aufblasen im Bereich der Konen und dickwandigen Enden Wandmaterial zur Wandstärkenverringerung mechanisch abgetragen wird. Hierzu muss besonders darauf geachtet werden, dass dieser Bearbeitungsschritt unterhalb der Glasübergangstemperatur des thermoplastischen Kunststoffmaterials vorgenommen wird. Insgesamt ist also die Herstellung solcher Ballons, wie sie aus US 7,217,278 B2 bekannt sind, herstellungstechnisch aufwändig.

Der Erfindung liegt die Aufgabe zugrunde, ein Verfahren zur Herstellung eines Ballonkatheters anzugeben, bei denen auf konstruktiv einfache Weise die beim Stand der Technik vorhandenen Verdickungen und Versteifungen aufgrund der in Längsfalten gelegten Konen zumindest am distalen, also äußersten Ende des Ballons vermieden werden.

Diese Aufgabe wird in verfahrenstechnischer Hinsicht durch die Merkmale des Anspruches 1 gelöst. Das Lösungsprinzip sieht dabei vor, den Ballon zumindest an einem Ende konenlos auszuführen, so dass er über eine große Länge eine unverändert dünne Wandstärke aufweist. Der Ballon wird über seine gesamte Länge in undilatiertem Zustand in Längsfalten gelegt, die sich in die Befestigungszone zumindest am distalen Ende des Ballonkatheters hineinerstrecken. Dort ist der Ballon durch die Längsfalten hindurch fluiddicht auf dem distalen Endbereich des Innenschaftes befestigt, so dass sich keinerlei konenbedingte Verdickung oder Versteifung am distalen Ende des Katheters zeigt. Der Ballons wird dabei mit Konen an seinem proximalen und distalen Ende mittels Fluiddruckbeaufschlagung aus einem plastisch reckbaren Kunststoffröhrchen hergestellt und der Konus am distalen Ende mittels Abschneides entfernt.

Vorteilhafterweise ist der Ballon an seinen beiden Enden - wie vorstehend erörtert - konenlos ausgebildet und dann entsprechend auch am distalen Ende des Außenschaftes über die Längsfalten hinweg fluiddicht am Außenschaft befestigt.

Erkennbar weist ein solcher Ballon trotz der Faltung auf seiner gesamten Länge keinerlei Verdickung im Durchmesser oder Versteifung durch größere Wandstärken auf. Ein solcher Ballonkatheter lässt sich also mit seinem distalen, mit dem Ballon versehenen Ende durch sehr enge Gefäße und um enge Biegungen in einem Gefäßsystem problemlos führen.

Gemäß einer bevorzugten Ausführungsform erfolgt die Befestigung des konenlosen Ballons mit seinen Längsfalten in den Befestigungszonen mittels eines Schrumpfschlauches unter thermoplastischer Verformung, so dass der Ballon mit seinen Längsfalten fluiddicht auf den Außen- bzw. Innenschaft des Katheters aufgeschweißt wird.

Weitere bevorzugte Ausführungsformen der Erfindung beziehen sich auf einen Einschnürring, der das jeweilige Ballonende vor seiner Befestigungszone einschnürt und damit die Verschweißung oder Verklebung in der Befestigungszone vor einer schälenden Belastung schützt. Damit wird einem Versagen des Ballons bei einer Druckbeauschlagung zum Dilatieren zuverlässig vorgebeugt.

Eine weitere Stabilisierung des Ballons an seinen Enden kann durch eine zum Ballonende hin jenseits der Einschnürung angelegte Gegendruckkammer erreicht werden, die den Ballon im Bereich seiner Fixierung und Einschnürung im Gleichgewicht hält.

Das distale Ende des Ballons kann gegebenenfalls mit dem Einschnürring an einem am distalen Ende des Innenschaftes sitzenden Spitzenkegel angeordnet sein, mit dessen Hilfe die Einführbarkeit des Katheters in Körpergefäße erheblich vereinfacht werden kann.

Einer weiteren Verbesserung der Dichtigkeit und Druckfestigkeit des dilatierbaren Ballons dient eine nach innen eingestülpte Anbringung der Ballonenden am Außen- oder Innenschaft. Eine solche "nach innen überrollte Konfiguration" ist bei der Dilatation des Ballons selbstdichtend. Die Schweiß- oder Klebenaht wird beim Dilatieren des Ballons dann auch nur auf Kompression belastet.

Schließlich kann eine alternative Einschnürung des dilatierbaren Ballons durch Verdrillungen im Bereich vor den Befestigungszonen des Ballons gebildet werden. Auch hier wird die Verbindung des Ballons zum jeweiligen Schaft hin vor schälender Belastung geschützt.

In verfahrenstechnischer Hinsicht wird die oben erwähnte Aufgabe durch Herstellungsmethoden gelöst, wie sie in den Verfahrensansprüchen näher angegeben sind. Zur Vermeidung unnötiger Wiederholungen ergeben sich die entsprechenden Produktionsprozesse mit Merkmalen und Vorteilen aus der nachfolgenden Beschreibung von Ausführungsbeispielen, worauf hier ausdrücklich Bezug genommen wird. Diese Ausführungsbeispiele werden anhand der beigefügten Zeichnungen im Folgenden näher erläutert, in denen zeigen:
- Fig. 1 bis 3: schematische Längsschnitte des distalen Endbereiches eines Ballonkatheters in verschiedenen Produktionsschritten,
- Fig. 4: einen Radialschnitt des Ballonkatheters gemäß Schnittlinie IV-IV nach Fig. 2,
- Fig. 5: eine schematische Längsansicht eines Ballonrohlings,
- Fig. 6 und 7: teilweise Längsaxialschnitte eines Ballonkatheters in einer alternativen Ausführungsform in zwei aufeinanderfolgenden Fertigungszwischenschritten im Bereich des distalen Ballonendes,
- Fig. 8: eine teilweise Seitenansicht eines Ballonkatheters an dessen distalem Ende mit einem beiderseits konenlosen Ballon in undilatiertem Zustand,
- Fig. 9 und 10: teilweise Längsaxialschnitte des Ballonkatheters an seinem distalen Ende in undilatiertem und dilatiertem Zustand des Ballons,
- Fig. 11: einen teilweisen Längsaxialschnitt eines Ballonkatheters in einer weiteren alternativen Ausführungsform,
- Fig. 12 und 13: Radialschnitte des Ballonkatheters gemäß den Schnittlinien XII-XII bzw. XIII-XIII nach Fig. 11,
- Fig. 14 bis 16: schematische Längsschnitte des distalen Endbereichs eines Ballonkatheters in Ausführungsformen mit Einschnürringen,
- Fig. 17: einen schematischen Axialschnitt eines Einschnürrings,
- Fig. 18: einen schematischen Längsschnitt des distalen Endbereichs eines Ballonkatheters mit selbstdichtenden, eingestülpten Ballonenden, sowie
- Fig. 19: einen schematischen Längsschnitt des distalen Endbereiches eines Ballonkatheters mit verdrillten Ballonenden.

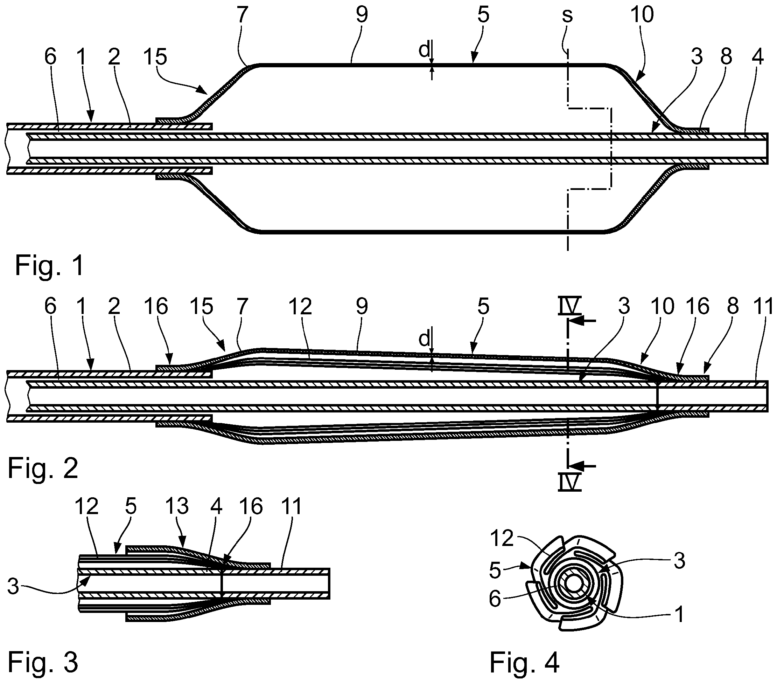

Anhand der Figuren 1 bis 4 werden die Herstellung und der Aufbau eines Ballonkatheters in einer ersten Ausführungsform näher erläutert. Ausgegangen wird dabei von einem im Wesentlichen zweiteiligen Katheterschaft, gebildet aus einem röhrenförmigen Außenschaft 1 mit einem distalen Ende 2 und einem gegenüber dessen Innendurchmesser im Außendurchmesser deutlich kleineren Innenschaft 3, dessen distales Ende 4 um mindestens die Länge des Ballons 5 über das distale Ende 2 des Außenschaftes 1 hinaussteht. Zwischen Außen- und Innenschaft 1, 3 ist eine ringförmige Fluidleitung 6 gebildet, über die ein entsprechendes Fluid unter Druck in den Ballon 5 geleitet werden kann.

Fig. 1 zeigt das distale Ende des Ballonkatheters in einem Fertigungszwischenschritt, bei dem ein plastisch umformbares Röhrchen der Länge des Ballons 5 mit seinem proximalen Ende 7 am distalen Ende 2 des Außenschaftes 1 und mit seinem distalen Ende 8 auf dem distalen Ende 4 des Innenschaftes 3 druckdicht befestigt wurde. Durch Einleiten eines Fluids ist dieses Röhrchen in die in Fig. 1 gezeigte Konfiguration mit dem zwischen proximalen und distalen Ende 7, 8 aufgeblasenen Ballon 5 gebildet. Die Mantelwand 9 des Ballons 5 ist dadurch mit einer sehr dünnen Wandstärke d ausgebildet, die in Richtung zum proximalen bzw. distalen Ende 7, 8 hin über den so gebildeten Konus 10, 15 beispielsweise um den Faktor 10 zunimmt. Damit ist insbesondere am distalen Ende 8 der Ballonkatheter mit einer relativ dicken Wulst versehen. Der so hergestellte Ballon wird ausgehend vom Konus 15 zum proximalen Ende 7 hin eng um den Innenschaft 3 in Längsfalten 12 gelegt, wie dies aus Fig. 4 besonders deutlich hervorgeht.

Um die oben erwähnte Wulst zu entfernen, wird an der in Fig. 1 angedeuteten Schnittlinie s der Ballon 5 und der Innenschaft 3 abgeschnitten. Damit wird auch der Konus 10 des Ballons 5 zu seinem distalen Ende 8 hin mit entfernt. Der Ballon 5 ist damit dort konenlos.

Wie aus Fig. 2 deutlich wird, wird dann der Innenschaft 3 an seinem distalen Ende mit Hilfe des gleichen Durchmesser und Wandstärke aufweisenden Verlängerungsstückes 11 gleichen oder unterschiedlichen Materials im Wesentlichen wieder auf seine ursprüngliche Länge gebracht.

Um den eng um Innenschaft 3 und Verlängerungsstück 11 herumgelegten Ballon 5 wird ein kurzes Schrumpfschlauchstück 13 gelegt. Dieser wird radial von außen mit nicht näher dargestellten ringförmigen beheizten Schweißbacken beaufschlagt, so dass das Schrumpfschlauchstück 13 radial geschrumpft und der gefaltete Ballon 5 an seinem distalen Ende auf den Innenschaft 3 und das Verlängerungsstück 11 gepresst und durch die Einwirkung der Hitze mit diesen Komponenten verschweißt wird. Anschließend kann das Schrumpfschlauchstück 13 wieder abgenommen werden.

Zusammenfassend ist der Ballon 5, wie er gemäß den Fig. 1 bis 4 hergestellt wird, an seinem proximalen Ende 7 in einer Befestigungszone 14 am Außenschaft 1 festgelegt und weist einen Konus 15 als Übergang zur Mantelwand 9 hin aus. Von dort aus ist der Ballon 5 mit über seine Länge unverändert dünner Wandstärke d ausgestaltet, die Längsfalten 12 erstrecken sich zum distalen Ende 8 des Ballons 5 hin konenlos in die dortige Befestigungszone 16 hinein.

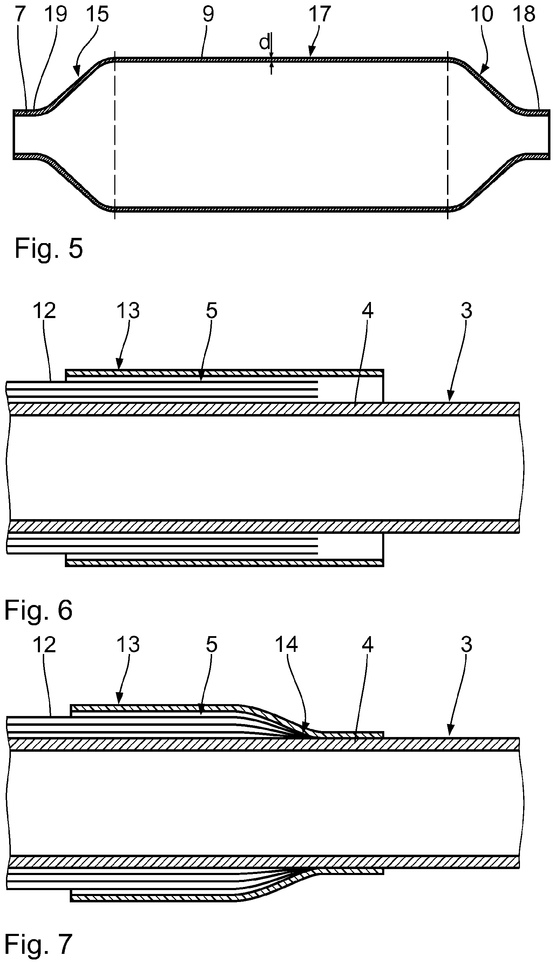

Anhand der Fig. 5 bis 10 ist nun ein Ballonkatheter in einer alternativen Ausführungsform in seinem Aufbau und seiner Herstellung zu erläutern, der an seinen beiden Enden 7, 8 konenlos ausgebildet ist.

Ausgegangen wird dabei von einem in üblicher Weise hergestellten Ballonrohling 17 mit dickwandigen Endstücken 18, 19 und Konen 10, 15. Wie dabei in Fig. 5 nicht angedeutet ist, kann der dargestellte Ballonrohling 17 auch mit einer solchen Gesamtlänge hergestellt werden, die einem Vielfachen der Länge eines Ballons 5 entspricht. Aus einem solchen Ballonrohling können dann, nachdem der Ballonrohling 17 gefaltet wurde, durch Ablängen mehrere konenlose Ballons 5 zur Weiterverarbeitung entstehen.

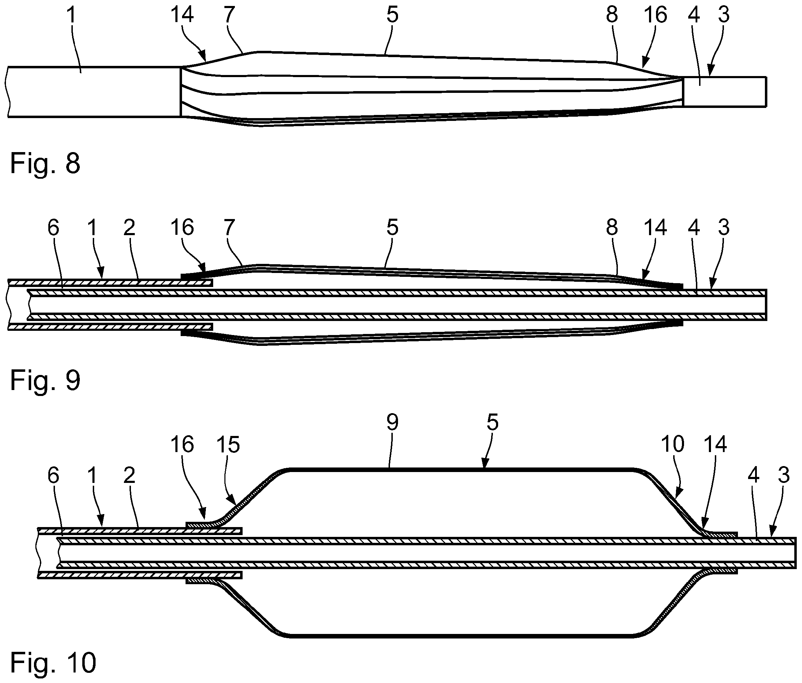

Wie aus Fig. 6 deutlich wird, wird nun für die Herstellung eines Ballonkatheters mit an beiden Enden konenlosem Ballon 5 der aus dem Ballonrohling 17 ausgeschnittene Ballon 5 wiederum in Längsfalten 12 um das distale Ende 2, 4 von Außen- und Innenschaft 1, 3 gelegt. In Fig. 6 ist lediglich das distale Ende 4 des Innenschaftes 3 gezeigt, die Konfiguration am Außenschaft 1 entspricht diesem Bild in spiegelverkehrter Darstellung. Wie in Fig. 5 durch gestrichelte Linien angedeutet ist, werden dann die Konen 10, 15 mit ihren Endstücken 18, 19 abgeschnitten, so dass lediglich die dünne Mantelwand 9 von dem Ballonrohling 17 übrig bleibt. Um das distale Ende 8 des Ballons 5 wird genauso wie um dessen proximales Ende (nicht dargestellt) ein Schrumpfschlauchstück 13 gelegt, das wieder mit Heißbacken radial beaufschlagt wird. Dadurch wird der Ballon 5 an seinen beiden Enden 7, 8 fluiddicht mit dem Innen- 3 bzw. Außenschaft 1 thermoplastisch verschweißt, wie dies in Fig. 7 dargestellt ist. Nach dem Abkühlen der somit hergestellten thermoplastischen Schweißverbindung zwischen Ballon 5 und Außen- 1 bzw. Innenschaft 3 wird das Schrumpfschlauchstück 13 wieder entfernt, so dass der Ballonkatheter an seinem distalen Ende die in Fig. 8 und 9 gezeigte Gestalt annimmt. Die durchgehend dünnwandige Mantelwand 9 des Ballons 5 ist in den beiden Befestigungszonen 14, 16 unter Einschluss der Längsfalten 12 fluiddicht thermoplastisch mit dem distalen Ende 2 des Außenschaftes 1 bzw. dem distalen Ende 4 des Innenschaftes 3 verschweißt. Der Ballon 5 bleibt an diesen kritischen Stellen somit in seinem Außendurchmesser optimal gering und zeigt keine Versteifungen.

Beim Dilatieren dieses Ballons 5 durch Einleiten eines unter Druck stehenden Fluids in die Fluidleitung 6 wird der Ballon aufgepumpt, wie dies in Fig. 10 angedeutet ist. Es bilden sich dabei durch Entfalten der Längsfalten 12 Konen 10, 15 am Ballon 5 aus, die jedoch genauso dünnwandig wie die Mantelwand 9 des Ballons 5 sind.

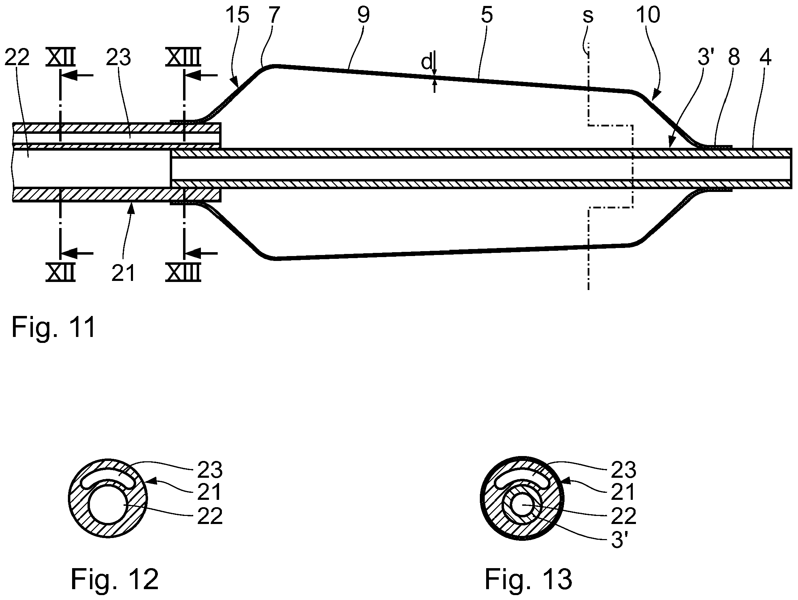

In den Fig. 11 bis 13 ist eine weitere alternative Ausführungsform dargestellt. Bei dieser weist der Katheter einen Zwei-Lumen-Schaft 21 auf, der ein kreisförmiges, etwas exzentrisch angeordnetes Drahtlumen 22 und ein im Querschnitt etwa bananenförmiges Ballonlumen 23 aufweist. Durch das Drahtlumen 22 ist ein Führungsdraht für den Ballonkatheter durchschiebbar, durch das Ballonlumen 23 kann das Fluid zum Dilatieren des Ballons 5 geführt werden. Das Drahtlumen kann als kurzes, sich nur über eine Teillänge des Katheters erstreckendes Lumen für eine sogenannte "Monorail-Variante" oder als vollständig durchgehendes Lumen für eine sogenannte "Over-the-wire-Variante" ausgelegt sein. Wie aus Fig. 11 deutlich wird, weist der Zwei-Lumen-Schaft 21 als Außenschaft keinen Innenschaft im Sinne der Ausführungsbeispiele gemäß den Fig. 1 bis 10 auf, vielmehr ist ein Innenschaft 3' durch Ansetzen eines kurzen Stückes an das Drahtlumen 22 des Zwei-Lumen-Schaftes 21 realisiert. Der Ballon 5 wird dann analog dem Ausführungsbeispiel gemäß den Fig. 1 bis 4 mit einem Ballon 5 am distalen Ende 2 des Zwei-Lumen-Schaftes 21 und am distalen Endes 4 des kurzstückigen Innenschaftes 3' verschweißt, in Falten gelegt, am distalen Ende 8 mit dem Konus 10 abgeschnitten und mit einem in Fig. 11 nicht dargestellten Verlängerungsstück komplettiert, um die in Fig. 2 bis 4 dargestellte Ballonkonfiguration an dessen distalen Ende zu erzielen.

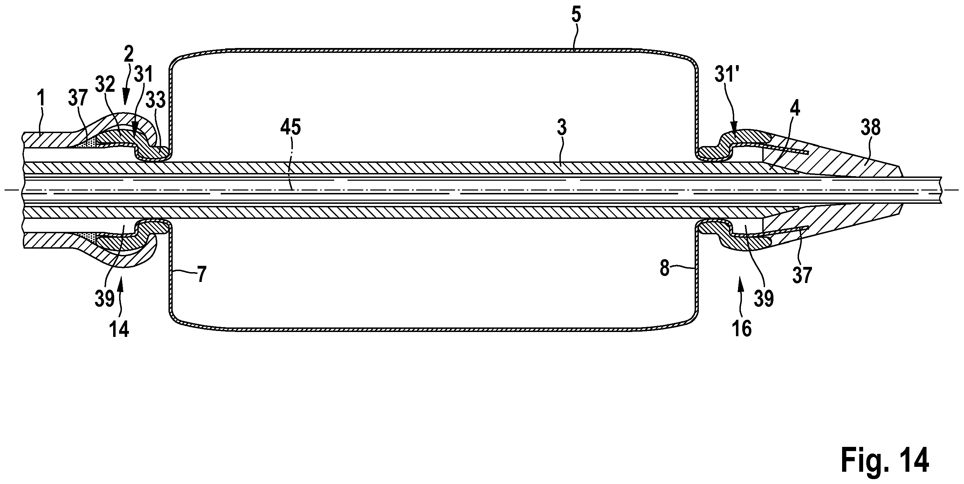

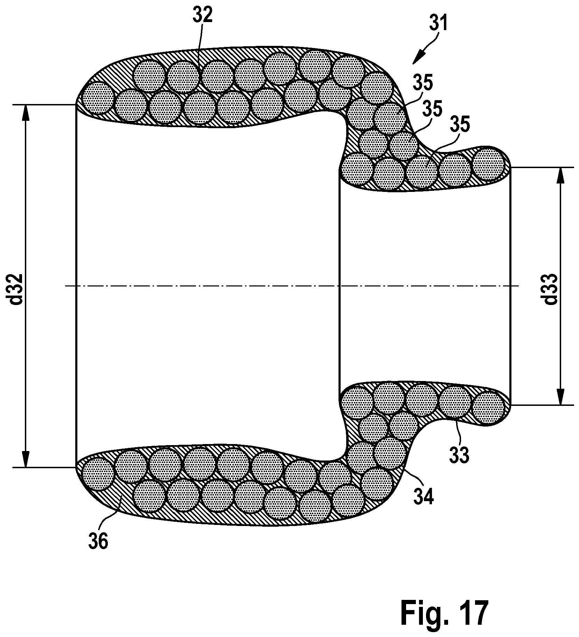

Bei der in Fig. 14 gezeigten Ausführungsform des Ballonkatheters ist am distalen Ende 2 des Außenschaftes 1 ein Einschnürring 31 eingesetzt, der-wie insbesondere aus Fig. 17 deutlich wird - einen Längsabschnitt 32 mit weitem Durchmesser d32 und einen Längsabschnitt 33 mit einem deutlich geringerem Durchmesser d33 aufweist. Beide Längsabschnitte 32, 33 sind über eine Stufenschulter 34 verbunden. Der Einschnürring 31 besteht in dieser Ausführungsform aus einem Fasergelege 35, das in einer Fixiermatrix 36 aus einem geeigneten polymeren Bindemittel sitzt. Der Einschnürring kann auch ein einfacher Ring aus einem hochfesten Material sein, der dann in einem Schlauch z.B. dem Außenschaft positioniert wird.

Wie aus Fig. 14 hervorgeht, ist das proximale Ende 7 des Ballons innen über den engen Längsabschnitt 33 des Einschnürrings 31 geführt und jenseits des weiten Längsabschnittes 32 in seiner Befestigungszone 14 mit einer Verbindung 37 an der Innenseite des Außenschaftes 1 befestigt. Bei der Verbindung 37 kann es sich um eine Verschweißung oder Verklebung handeln.

Am distalen Ende 8 ist der Ballon 5 gleichermaßen durch einen Einschnürring 31' geführt, dessen enger Längsabschnitt 33 dem dilatierbaren Ballonvolumen zugewandt ist. Das Ballonende ist darauffolgend zwischen einem auf dem distalen Ende 4 des Innenschaftes 3 sitzenden Spitzenkegel festgelegt.

Die im Bereich des weiten Längsabschnittes 32 liegenden Passagen der Ballonenden 7, 8 werden beim Dilatieren des Ballons ebenfalls dilatiert und bilden somit Gegendruckkammern 39, die für eine Stabilisierung des Ballons 5 in der durch den jeweiligen Einschnürring 31, 31' gebildeten Führung sorgen und damit eine zuverlässigen, druckdichten Befestigung des Ballons 5 unterstützen.

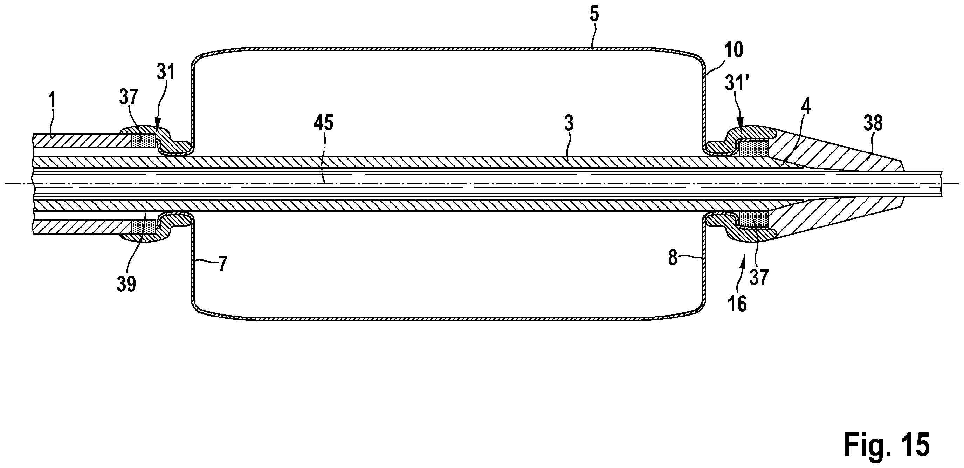

Während bei der Ausführungsform gemäß Fig. 14 der Einschnürring 31 mit seinem weiten Längsabschnitt 32 in den etwas aufgeweiteten Außenschaft 1 eingepresst und dort befestigt ist, wird bei der Ausführungsform gemäß Fig. 15 der proximale Einschnürring 31 auf den Außenschaft 1 gesetzt und das proximale Ende 7 des Ballons 5 über die Schweiß- oder Klebe-Verbindung 37 stumpf mit dem Außenschaft 1 verbunden. Das proximale Ende 7 des Ballons 5 ist wiederum durch den Einschnürring 31 geführt, wodurch die Verbindung 37 vor schälender Belastung geschützt ist. Es ist eine Gegendruckkammer 39 auf der Seite des weiten Längsabschnittes 32 des Einschnürrings 31 angelegt.

Das distale Ende 8 des Ballons 5 ist ebenfalls durch einen Einschnürring 31' hindurchgeführt, der mit seinem weiten Längsabschnitt 32 über eine Verbindung 37 auf dem distalen Ende 4 des Innenschaftes 3 vor dem Spitzenkegel 38 befestigt ist. Dabei ist das distale Ende 8 des Ballons 5 in die Verbindung 37 mit eingebettet.

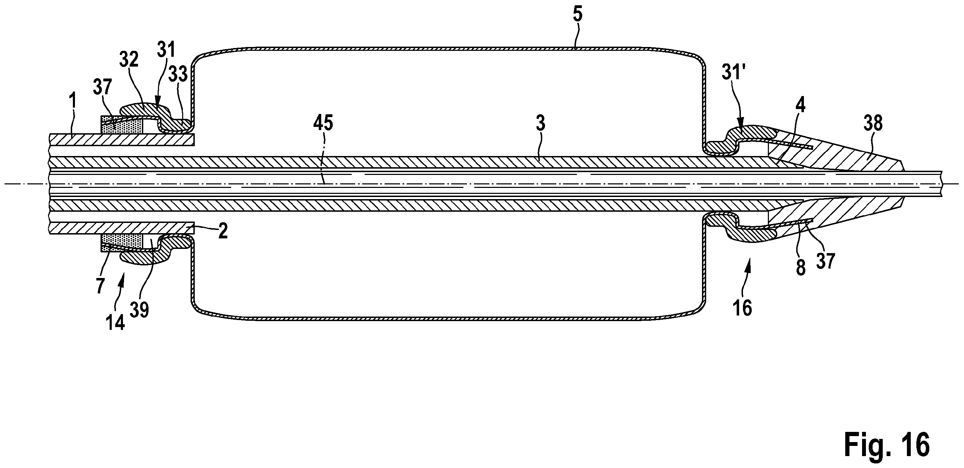

Bei der Ausführungsform gemäß Fig. 16 wird ein proximaler Einschnürring 31 außen auf den Außenschaft 1 an dessen distales Ende 2 gesetzt und über eine Verbindung 37 befestigt. In dieser ist gleichzeitig das proximale, durch den Einschnürring 31 geführte Ende 7 des Ballons festgelegt. Zwischen Verbindung 37 und Einschnürring 31 ist im Bereich des weiten Längsabschnittes 32 wiederum eine Gegendruckkammer 39 ausgebildet. Insgesamt ist das proximale Ende des Ballons 5 in seiner Befestigungszone 14 durch den Einschnürring 31 vor schälender Belastung geschützt.

Die Verankerung des distalen Endes 8 des Ballons 5 entspricht der Ausführungsform gemäß Fig. 14, sodass sich eine nochmalige Erörterung erübrigt.

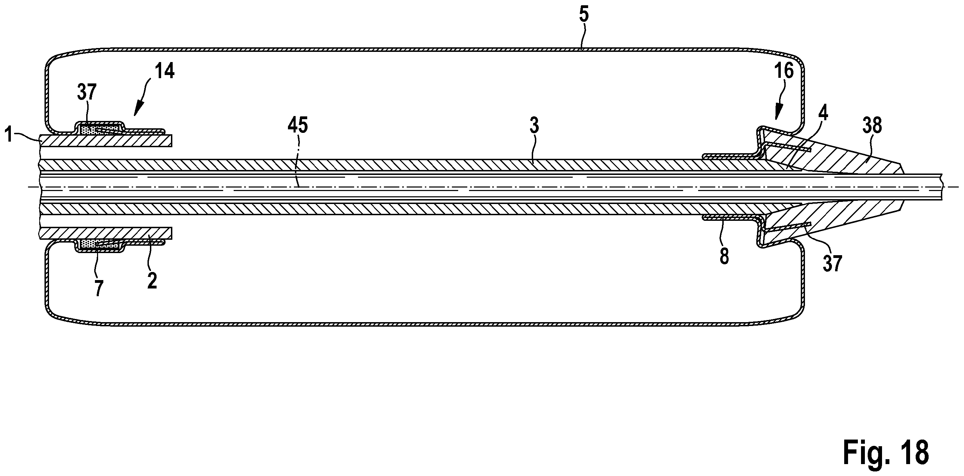

Bei der in Fig. 18 gezeigten Ausführungsform sind die proximalen und distalen Enden 7, 8 des Ballons 5 nach innen eingestülpt und über entsprechende Verbindungen 37 auf dem distalen Ende 2 des Außenschaftes 1 beziehungsweise dem distalen Ende 4 des Innenschaftes 3 festgelegt. Die entsprechenden Schweiß- oder Klebenähte der Verbindung 37 sind durch dieses nach innen überrollte Anlegen des Ballons 5 bei dessen Dilatieren nur auf Kompression belastet. Zu beachten ist, dass die Enden 7, 8 des Ballons 5 im eingestülpten Bereich in zwei Lagen auf sich selbst gelegt sind. Insgesamt wird durch die gezeigte Konfiguration gemäß Fig. 18 ein konenloser Ballon mit selbstdichtenden Befestigungszonen 14, 16 am Außen- und Innenschaft 3 erreicht.

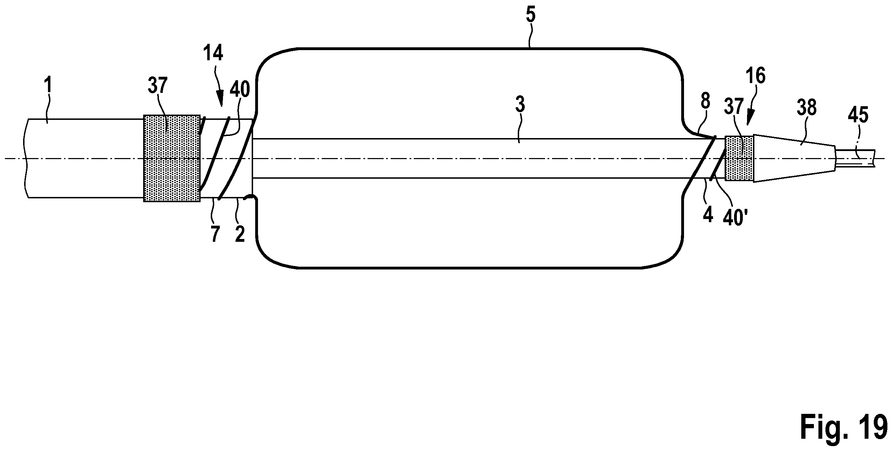

In Fig. 19 ist eine weitere Ausgestaltung eines Ballonkatheters gezeigt, bei dem ein konenloser Ballon 5 an seinen beiden Enden 7, 8 jeweils durch gleichsinnige Verdrillungen 40, 40' vor der eigentlichen proximalen und distalen Befestigungszone 14, 16 eingeschnürt ist. In diesen beiden Befestigungszonen 14, 16 ist jeweils wiederum eine Verbindung 37 in Form einer Verschweißung oder Verklebung des Ballons 5 auf dem Außenbeziehungsweise Innenschaft 1, 3 vorgesehen. Damit ist abermals die Verbindung des Ballons mit dem jeweiligen Schaft vor schälender Belastung geschützt. Damit sich die Ballonenden 7, 8 nicht entdrillen, sind Innen- und Außenschaft 1, 3 drehfest zueinander zu fixieren.

In allen Ausführungsformen gemäß Fig. 14 bis 19 ist im Lumen des Innenschaftes 3 ein Führungsdraht 45 für den Katheter gezeigt.

## Patentansprüche

1. Verfahren zur Herstellung eines Ballonkatheters umfassend
- einen Außenschaft (1) mit einem distalen Ende (2) und einer Fluidleitung (6),
- einen zumindest an dessen distalem Ende (2) angeordneten Innenschaft (3), der über das distale Ende (2) des Außenschaftes (1) hinaussteht, sowie
- einen unter Einfluss eines durch die Fluidleitung (6) unter Druck eingebrachten Fluids dilatierbaren Ballon (5), der
= mit einer ersten Befestigungszone (16) an seinem proximalen Ende (7) fluiddicht auf dem distalen Endbereich (2) des Außenschaftes (1),
= mit einer zweiten Befestigungszone (14) an seinem distalen Ende (8) fluiddicht auf dem distalen Endbereich (4) des Innenschaftes (3) befestigt ist und
= zwischen den Befestigungszonen (14, 16) auf seiner Länge in undilatiertem Zustand in Längsfalten (12) gelegt ist,
**gekennzeichnet durch** folgende Verfahrensschritte:
- Herstellen eines Ballonrohlings (17) mit Konen (10, 15) an seinem proximalen und distalen Ende (7, 8) mittels Fluiddruckbeaufschlagung aus einem plastisch reckbaren Kunststoffröhrchen, wobei die Enden des Röhrchens in eine Haltevorrichtung gespannt werden, worauf das Lumen mit einem Fluiddruck beaufschlagt wird, wodurch das Werkstück zwischen den Einspannpunkten aufgeblasen und das Wandmaterial gereckt wird, so dass ein zylindrischer Ballon entsteht,
- Legen des Ballons (5) in Längsfalten (12) um das distale Ende (2, 4) von Außen- und Innenschaft (1, 3),
- Abschneiden der Konen (10, 15) am proximalen und distalen Ende (7, 8) des Ballonrohlings (17), und
- fluiddichtes Aufschweißen der in Längsfalten (12) gelegten proximalen und distalen Endes (7, 8) des Ballons (5) auf den Außen- und Innenschaft (1, 3) des Katheters, der Ballon (5) an seinem proximalen und distalen Ende (7, 8) konenlos mit über seine Länge unverändert dünner Wandstärke (d) ausgestaltet ist und sich die Längsfalten (12) in die dortige Befestigungszone (14) hineinerstrecken.

2. Verfahren nach Anspruch 1, **dadurch gekennzeichnet, dass** das Aufschweißen der in Längsfalten (12) gelegten Enden (7, 8) des Ballons (5) auf den Außen- und/oder Innenschaft (1, 3) des Katheters mit Hilfe eines dort um die Längsfalten (12) gelegten Schrumpfschlauches (13) erfolgt.

3. Verfahren nach Anspruch 1, **dadurch gekennzeichnet, dass** der an seinen beiden Enden mit Konen (10, 15) versehene Ballonrohling (17) in einer Gesamtlänge hergestellt wird, die einem Vielfachen der Länge eines Ballons (5) entspricht, wobei aus diesem Ballonrohling (17) durch Ablängen die konenlosen Ballons (5) zur Weiterverarbeitung entstehen.

4. Verfahren nach einem der vorgenannten Ansprüche, **dadurch gekennzeichnet, dass** der Ballon (5) vor seiner proximalen und/oder distalen Befestigungszone (14, 16) mit einem Einschnürring (31, 31') beaufschlagt wird.

5. Verfahren nach Anspruch 4, **dadurch gekennzeichnet, dass** der Einschnürring (31, 31') durch ein in eine Fixiermatrix (36) eingebettetes Fasergelege (35) gebildet wird.

6. Verfahren nach Anspruch 4 oder 5, **dadurch gekennzeichnet, dass** auf der dem Ballonvolumen abgewandten Seite des Einschnürrings (31, 31') eine Gegendrucckammer (39) im proximalen und/oder distalen Ende (7, 8) des Ballons (5) angelegt wird.

7. Verfahren nach einem der vorgenannten Ansprüche, **dadurch gekennzeichnet, dass** der Ballon (5) zumindest in seiner proximalen Befestigungszone (14) innen oder stumpf am Außenschaft (1) befestigt wird.

8. Verfahren nach einem der Ansprüche 1 bis 4, **dadurch gekennzeichnet, dass** das proximale und/oder distale Ende (7, 8) des Ballons (5) nach innen eingestülpt am Außen- bzw. Innenschaft (1, 3) befestigt wird oder dass der Ballon (5) an seinem proximalen und distalen Ende (7, 8) durch jeweils eine innerhalb der Befestigungszone (14, 16) angeordnete Verdrillung (40, 40') gleichen Drehsinns eingeschnürt wird.

9. Verfahren nach einem der vorgenannten Ansprüche, **dadurch gekennzeichnet, dass** das distale Ende (8) des Ballons (5) oder gegebenenfalls der Einschnürring (31') an einem am distalen Ende des Innenschaftes (3) sitzenden Spitzenkegel (38) angeordnet wird.

## Claims

1. A method for producing a balloon catheter, comprising
- an outer shaft (1) having a distal end (2) and a fluid line (6),
- an inner shaft (3), which is arranged at least at the distal end (2) of said outer shaft and which protrudes beyond the distal end (2) of the outer shaft (1), and
- a balloon (5), which can be dilated under the influence of a fluid introduced under pressure through the fluid line (6) and which
= is fastened at its proximal end (7) in a fluid-tight manner to the distal end region (2) of the outer shaft (1) by means of a first fastening zone (16),
= is fastened at its distal end (8) in a fluid-tight manner to the distal end region (4) of the inner shaft (3) by means of a second fastening zone (14), and
= between the fastening zones (14, 16), in the undilated state, is placed in longitudinal folds (12) over its length,
**characterised by** the following method steps:
- producing a balloon blank (17) having cones (10, 15) at its proximal and distal end (7, 8) from a plastically stretchable plastics tube by application of fluid pressure, wherein the ends of the tube are clamped in a holding device, whereupon the lumen is subjected to a fluid pressure, whereby the workpiece is inflated between the clamping points and the wall material is stretched, such that a cylindrical balloon is formed,
- placing the balloon (5) in longitudinal folds (12) around the distal end (2, 4) of the outer and inner shaft (1,3),
- cutting off the cones (10, 15) at the proximal and distal end (7, 8) of the balloon blank (17), and
- welding the proximal and distal end (7, 8) of the balloon (5) placed in longitudinal folds (12) onto the outer and inner shaft (1, 3) of the catheter in a fluid-tight manner, the balloon (5) being cone-free at its proximal and distal end (7, 8), with the same thin wall thickness (d) over its length, and the longitudinal folds (12) extending into the fastening zone (14) there.

2. The method according to claim 1, **characterised in that** the ends (7, 8) of the balloon (5) placed in longitudinal folds (12) are welded onto the outer and/or inner shaft (1, 3) of the catheter with the aid of a shrink tube (13) placed there around the longitudinal folds (12).

3. The method according to claim 1, **characterised in that** the balloon blank (17) provided at its two ends with cones (10, 15) is produced in an overall length corresponding to a multiple of the length of a balloon (5), wherein the cone-free balloons (5) for further processing are produced from this balloon blank (17) by cutting to length.

4. The method according to any one of the preceding claims, **characterised in that** the balloon (5) is acted on with a constriction ring (31, 31') in front of its proximal and/or distal fastening zone (14, 16).

5. The method according to claim 4, **characterised in that** the constriction ring (31, 31') is formed by a laid fibre fabric (35) embedded in a fixing matrix (36).

6. The method according to claim 4 or 5, **characterised in that** a counter pressure chamber (39) in the proximal and/or distal end (7, 8) of the balloon (5) is arranged on the side of the constriction ring (31, 31') remote from the balloon volume.

7. The method according to any one of the preceding claims, **characterised in that** the balloon (5), at least in its proximal fastening zone (14), is fastened to the outer shaft (1) on the inside or is fastened thereto by means of a butt joint.

8. The method according to any one of claims 1 to 4, **characterised in that** the proximal and/or distal end (7, 8) of the balloon (5) is fastened to the outer or inner shaft (1, 3) in a manner drawn inwardly thereinto, or **in that** the balloon (5) at each of its proximal and distal end (7, 8) is constricted by a twisted portion (40, 40') arranged within the fastening zone (14, 16), said twisted portions having the same twist direction.

9. The method according to any one of the preceding claims, **characterised in that** the distal end (8) of the balloon (5) or as applicable the constriction ring (31') is arranged on a pointed cone (38) sitting at the distal end of the inner shaft (3).

## Revendications

1. Procédé de fabrication d'un cathéter à ballonnet comprenant
- une tige extérieure (1) avec une extrémité distale (2) et une conduite de fluide (6),
- au moins une tige intérieure (3), disposée à son extrémité distale (2), qui dépasse par-dessus l'extrémité distale (2) de la tige extérieure (1), ainsi
- qu'un ballonnet (5) dilatable sous l'influence d'un fluide mis sous pression introduit à travers la conduite de fluide (6), qui
= est fixé de manière étanche aux fluides par une première zone de fixation (16) à son extrémité proximale (7) sur la zone d'extrémité distale (2) de la tige extérieure (1),
= est fixé de manière étanche aux fluides par une seconde zone de fixation (14) à son extrémité distale (8) sur la zone d'extrémité distale (4) de la tige intérieure (3), et
= est posé entre des zones de fixation (14, 16) sur sa longueur dans un état non dilaté en plis longitudinaux (12),
**caractérisé par** les étapes de procédé suivantes :
- de fabrication d'une ébauche de ballonnet (17) avec des cônes (10, 15) à son extrémité proximale et à son extrémité distale (7, 8) au moyen d'une mise sous pression de fluide à partir d'un petit tube en matière plastique pouvant être extensible de manière plastique, où les extrémités du petit tube sont tendues dans un dispositif de maintien, à la suite de quoi la lumière est soumise à une pression de fluide, ce par quoi la pièce est gonflée entre les points de mise sous tension et le matériau de la paroi est étendu, de sorte qu'il se forme un ballonnet cylindrique,
- de mise du ballonnet (5) sous forme de plis longitudinaux (12) autour de l'extrémité distale (2, 4) de la tige extérieure et de la tige intérieure (1, 3),
- de découpe des cônes (10, 15) aux extrémités proximale et distale (7, 8) de l'ébauche de ballonnet (17), et
- de soudage de manière étanche aux fluides des extrémités proximale et distale (7, 8)) mis en plis longitudinaux (12) du ballonnet (5) sur les tiges extérieure et intérieures (1, 3) du cathéter, le ballonnet (5) est conçu exempt de cônes à ses extrémités (7, 8) avec une épaisseur de paroi (d) plus mince inchangée sur sa longueur et les plis longitudinaux (12) s'étendent à l'intérieur de la zone de fixation (14) à cet endroit.

2. Procédé selon la revendication 1,
**caractérisé en ce que** le soudage des extrémités (7, 8) mises en plis longitudinaux (12) du ballonnet (5) sur la tige extérieure et/ou la tige intérieure (1, 3) du cathéter a lieu à l'aide d'une gaine rétractable (13) mise en plis longitudinaux (12) à cet endroit.

3. Procédé selon la revendication 1,
**caractérisé en ce que** l'ébauche de ballonnet (17) munie de cônes (10, 15) à ses deux extrémités est fabriquée sur une longueur totale qui correspond à un multiple de la longueur d'un ballonnet (5), où les ballonnets (5) sans cônes sont créés pour être façonnés ultérieurement à partir de cette ébauche de ballonnet (17) par des tronçonnages.

4. Procédé selon l'une des revendications précitées,
**caractérisé en ce que** le ballonnet (5) est soumis à un anneau de serrage (31, 31') avant sa zone d'extrémité proximale et/ou distale (14, 16).

5. Procédé selon la revendication 4,
**caractérisé en ce que** l'anneau de serrage (31, 31') est formé par une structure de fibres (35) incorporée dans une matrice de fixation (36).

6. Procédé selon la revendication 4 ou la revendication 5,
**caractérisé en ce qu'**une chambre de pression opposée (39) est appliquée à l'extrémité proximale et/ou distale (7, 8) du ballonnet (5) sur le côté de l'anneau de serrage (31, 31') opposé au volume du ballonnet.

7. Procédé selon l'une des revendications précitées,
**caractérisé en ce que** le ballonnet (5) est fixé à l'intérieur ou mis bout à bout sur la tige extérieure (1) au moins dans sa zone de fixation (14) proximale.

8. Procédé selon l'une des revendications 1 à 4,
**caractérisé en ce que** l'extrémité proximale et/ou distale (7, 8) du ballonnet (5) est fixée sur la tige extérieure, respectivement, intérieure (1, 3), en étant repliée vers l'intérieur, ou que le ballonnet (5) est fixé par un lien au niveau de ses extrémités proximale et distales (7, 8) par un vrillage (40, 40') respectif dans le même sens de rotation, disposé à l'intérieur de la zone de fixation (14, 16).

9. Procédé selon l'une des revendications précitées,
**caractérisé en ce que** l'extrémité distale (8) du ballonnet (5) ou éventuellement l'anneau de fixation (31') est disposé sur un cône d'extrémité (38) situé à l'extrémité distale de la tige intérieure (3).
